# EUROPÄISCHE PATENTANMELDUNG

(11) **EP 1 258 257 A2**
(43) Veröffentlichungstag der Anmeldung: **20.11.2002**
(21) Anmeldenummer: 02009311.8
(22) Anmeldetag: 02.05.2002
(51) Int. Cl.: A61L 15/58, A61L 15/34

(54) **Hautpflegende Klebstoffzusammensetzung**

(30) Priorität: 15.05.2001 CH 9012001
(71) Anmelder: IVF Hartmann AG, 8212 Neuhausen am Rheinfall (CH)
(72) Erfinder: Mähr, Rodolfo, 8207 Schaffhausen (CH); Möller, Kathrin, 8200 Schaffhausen (CH)
(74) Vertreter: Blum, Rudolf Emil Ernst

(57) **Zusammenfassung**

Es wird eine Klebstoffzusammensetzung beschrieben, die ein Poly(meth)acrylat und mindestens ein fettes Öl umfasst, insbesondere Mandelöl. Eine solche Klebstoffzusammensetzung ist speziell geeignet für den Medizinalbereich, insbesondere für die Herstellung hautpflegender Pflaster.

## Beschreibung

Die vorliegende Erfindung betrifft hautpflegende Klebstoffzusammensetzungen, insbesondere hautpflegende Klebstoffzusammensetzungen für den Medizinalbereich sowie daraus hergestellte Verbandmaterialien.

Verbände über kleineren Wunden oder an für normale, nicht geklebte Verbände ungeeigneten Stellen werden heute oft mittels Pflaster befestigt. Zur Verabreichung von Wirkstoffen über und an die Haut werden u.a. sogenannte transdermale therapeutische Systeme (z.B. Hormonpflaster) in Pflasterform eingesetzt. Solche Pflaster haben den Nachteil, dass sie nach längerem oder wiederholtem Gebrauch zu Austrocknung der Haut, Hautrötungen, gelegentlich sogar zu vollständiger Unverträglichkeit führen. Mit der neueren Generation der Vliespflaster wurde zwar ein wesentlich hautfreundlicheres Verbandmaterial bereitgestellt, dennoch können nach längerem Gebrauch auch bei diesen Pflastern unerwünschte Reaktionen, wie Hauttrockenheit, Rötungen, Juckreiz etc. auftreten. Ferner gründet die gute Verträglichkeit der Vliespflaster primär auf der guten Luftdurchlässigkeit des Vlieses, die jedoch mit einem Verzicht auf Eigenschaften wie Elastizität oder Wasserabstossung verbunden ist.

Es besteht deshalb immer noch das Bedürfnis nach hautpflegenden klebbaren Verbandmaterialien mit unterschiedlichen Eigenschaften.

Ziel der vorliegenden Erfindung war es deshalb die Hautverträglichkeit von klebbaren Verbandsmaterialien und wirkstoffhaltigen Pflastern zu erhöhen.

Dieses Ziel wurde erreicht durch die Bereitstellung eines Klebstoffes gemäss Anspruch 1. Spezielle Ausführungsformen sind den abhängigen Ansprüchen und weitere Aspekte der Erfindung den weiteren unabhängigen Ansprüchen zu entnehmen.

Überraschenderweise wurde gefunden, dass Klebstoffen auf Acrylatbasis pflegende Öle, insbesondere Öle mit rückfettenden und hautberuhigenden Eigenschaften, beigegeben werden können ohne dass die Klebeeigenschaften nachteilig beeinflusst werden. In einigen Fällen wird die Klebkraft durch Zugabe von fetten Ölen sogar erhöht.

Die Klebstoffzusammensetzungen der vorliegenden Erfindung sind Kontaktklebstoffe sowie insbesondere druckempfindliche Klebstoffe. Aufgrund ihres verhältnismässig geringen allergenen Potentials und des günstigen Einbau- resp. Abgabeverhaltens sind Kontaktklebstoffe und druckempfindliche Klebstoffe auf Acrylatbasis bevorzugt.

Im Rahmen dieser Erfindung geeignete druckempfindliche Klebstoffe werden aus Polyacrylaten und Polymethacrylaten hergestellt. Homopolymere und Copolymere mit tiefem Glasumwandlungspunkt können eingesetzt werden um die gewünschte Klebrigkeit zu erzielen. Die Glasumwandlungstemperatur kann z.B. durch Copolymerisation mit Acrylonitril, Styrol, Vinylacetat oder Methylmethacrylat beeinflusst werden. Je höher der Glasumwandlungspunkt, desto tiefer ist im allgemeinen das Molekulargewicht, je höher das Molekulargewicht, desto besser die Kohäsion des Films. Für die Anwendung im Medizinalbereich müssen die Klebstoffe derart gewählt werden, dass deren Adhäsion ausreicht um während des gewünschten Zeitraums gute Haftung auf der Haut zu gewährleisten, wobei die Adhäsion am Träger aber höher sein sollte als die Adhäsion an der Haut, und die Adhäsion an der Haut sollte geringer sein als die Kohäsion, so dass beim Entfernen des Verbandmaterials ein Adhäsionsbruch zwischen der Haut und der Klebeschicht auftritt, derart, dass möglichst keine Rückstände auf der Haut zurückbleiben. Ferner müssen allenfalls enthaltene Lösungsmittel und Hilfsstoffe nach dem Aufbringen der flüssigen Schicht auf den Träger leicht entfernbar sein und der Rückstand sollte keine hautreizenden oder über die Haut in den Körper eindringenden schädlichen Stoffe enthalten. Geeignete Klebstoffe können auch organische oder anorganische Füllstoffe, z.B. Kautschuke und/oder Verdickungsmittel, enthalten oder unschädliche Additive zur Einstellung bestimmter Klebstoffeigenschaften. Bevorzugte Acrylatklebstoffe, die für den Medizinalbereich geeignet sind, sind bekannt und umfassen carboxylgruppenhaltige Acrylsäureestercopolymere z.B. mit Acrylnitril, sowie Polyacrylsäurealkylester, z.B. Butylester. Solche Acrylatklebstoffe sind unter dem Namen Acronal® von BASF Aktiengesellschaft, D-6700 Ludwigshafen erhältlich. Bevorzugte Produkte sind Acronal® 85 D, Acronal® A 107 und Acronal® V 205.

Einer solchen Acrylatmatrix kann ein hautpflegendes, insbesondere rückfettendes und/oder hautberuhigendes Öl beigemischt werden, insbesondere Mandelöl. Mandelöl ist bevorzugt, da es einen sehr ausgeprägten kosmetischen Effekt bewirkt und Hautirritationen rasch mildert. Ferner hat es sich überraschenderweise gezeigt, dass Mandelöl in ausreichender Menge an der Klebstoffoberfläche vorhanden ist, resp. aus der Klebstoffschicht an resp. in die Haut diffundiert, jedoch nicht in solchen Mengen resp. so schnell, dass sich auf der Haut ein öliger Film bilden würde, der die Klebeverbindung beeinträchtigen könnte.

Auch bei längerer Lagerung entsprechender Klebstoffschichten wurde keine unerwünscht hohe Diffusion des Öls, insbesondere des Mandelöls oder einzelner Bestandteile desselben, an die hautseitig zu applizierende Oberfläche beobachtet.

Das Öl kann in Mengen von bis zu 10 Gew.-% bezogen auf das Gewicht der getrockneten Klebstoffschicht eingebracht werden ohne dass die Haftung beeinträchtigt wird. Üblicherweise reichen aber Mengen von 3 bis 6 Gew.-%. Neben dem bevorzugten Mandelöl und dessen Komponenten kommen weitere pflegende Öle, insbesondere auch in der Kosmetik verwendete Öle aus der Gruppe der fetten Öle in Frage, wie beispielsweise Jojobaöl, Avocadoöl etc.

Eine solche ölhaltige Klebstoffzusammensetzung ist geeignet zur Herstellung einer Vielzahl von Pflastern und Verbänden durch Auftrag des Klebstoffs auf einen entsprechenden Träger.

Geeignete Träger sind beispielsweise Gewebe und Gewirke resp. Vliese, wie sie für die Herstellung elastischer Pflaster und Vliespflaster bekannt sind, aber auch Plastikfolien, wie sie bei der Herstellung wasserabstossender Pflaster Anwendung finden, oder mit elastischen Fäden durchwirkte Materialien, wie sie aus der Herstellung von Klebeverbänden, z.B. als nichtverrutschbare Stütze beim Sport, bekannt sind. Entsprechend hergestellte Pflaster können auch mit behandelter oder unbehandelter Gaze oder einem anderen Wundkissen, insbesondere einem Vlies, versehen werden, so dass diese als Schnellverband eingesetzt werden können.

Die Herstellung der Klebstoffzusammensetzung erfolgt dadurch, dass der Poly(meth)acrylatdispersion das Öl vor dem Aufbringen auf den Träger zugegeben wird.

Für die Verwendung in transdermalen therapeutischen Systemen enthält die Klebstoffzusammensetzung neben mindestens einem fetten Öl mindestens einen Wirkstoff und gegebenenfalls mindestens einen die Wirkstoffabgabe und/oder die Wirkstoffpenetration fördernden Stoff.

Eine solche Zusammensetzung kann ebenfalls durch einfaches Mischen aller Komponenten mit einem Dispergierer erhalten werden, wobei die Reihenfolge der Zugabe und der Zeitpunkt des Mischens (nach jeder einzelnen Zugabe oder am Schluss) üblicherweise unkritisch sind. Meist werden alle Komponenten zugegeben und anschliessend gemischt.

Transdermale therapeutische Systeme können für die Verabreichung einer Vielzahl von Wirkstoffen eingesetzt werden, z.B. für Hormone, Nikotin, entzündungshemmende und/oder schmerzlindernde Stoffe, wie Diclofenac, Morphin. Weitere Beispiele für Wirkstoffe sind Oleoresin, das schmerzlindernde Eigenschaften aufweist, Rosmarinöl, das durchblutungsfördernd ist, oder Prolin, das ebenfalls schmerzlindernd wirkt.

Stoffe, die die Wirkstoffabgabe und/oder die Penetration fördern sind beispielsweise Fettsäuren, Propylenglykol, Harnstoff, Glycerin etc.

Es liegt selbstverständlich auch im Rahmen der vorliegenden Erfindung der Klebstoffzusammnesetzung weitere Stoffe beizumischen, wie Duftstoffe, z.B. Lavendelöl oder Pfefferminzöl. Die Anwesenheit solcher Stoffe ist insbesondere vorteilhaft bei transdermalen therapeutischen Systemen mit unangenehm riechenden Wirkstoffen.

Zur Herstellung des klebenden Verbandmaterials oder des transdermalen therapeutischen Systems wird eine erfindungsgemässe Klebstoffzusammensetzung, d.h. eine ölhaltige Poly(meth)acrylatdispersion, in der gewünschten Menge auf einen Träger direkt oder im Transferverfahren (siehe unten) aufgebracht (für transdermale therapeutische Systeme üblicherweise in einer Menge von ca. 400 bis 600 g/m² bei einem Feststoffanteil von 50 bis 55 Gew-%, vorzugsweise ca. 550 g/m² bei einem Feststoffanteil von ca. 52 Gew.-%, und für Heftpflaster bzw. Schnellverbände üblicherweise in einer Menge von 100 bis 250 g/m², wobei sich die Mengen bei höherem Feststoffanteil von bis zu ca. 70 Gew.-% entsprechend reduzieren, resp. bei tieferem Feststoffgehalt entsprechend erhöhen) und anschliessend bei Temperaturen von bis zu 150°C, insbesondere bei Temperaturen von 85 bis 150°C, getrocknet. Dabei müssen bei transdermalen therapeutischen Systemen die Temperaturstabilität des Wirkstoffs und allfällige Verluste flüchtiger Wirkstoffe berücksichtigt werden.Die Trocknung kann beispielsweise mittels Heissluft erfolgen.

Geeignete Trägermaterialien wurden bereits vorne genannt. Als Schutzfolie oder -papier geeignet sind Materialien, die sich leicht entfernen lassen, wie schwach haftende Kunststoffolien und insbesondere mindestens klebstoffseitig silikonisierte Papiere, vorzugsweise einseitig Polyethylen (PE)-beschichtete Papiere mit Silikonisierung auf der PE-Schicht.

Im Transferverfahren, z.B. für die Herstellung eines transdermalen therapeutischen Systems, wird die Schutzfolie bzw. das Schutzpapier mit Klebstoff beschichtet, die Klebstoffschicht anschliessend mit Trägermaterial bedeckt und danach getrocknet. Gegebenenfalls kann die zur Beschichtung verwendete Folie bzw. das Papier in einem zweiten Arbeitsgang entfernt und durch eine für die Anwendung besser geeignete Schutzschicht ersetzt werden. Bei der Anwendung wird dann zuerst die Schutzschicht entfernt und dann der mit der Klebstoffschicht versehene Träger klebstoffseitig auf die Haut aufgebracht.

Bei Heftpflastern wird in der Regel der Träger direkt beschichtet und ohne Schutzschicht aufgerollt. Bei Bedarf, d.h. falls der Träger sonst auch rückseitig mit der Beschichtung verkleben würde, kann aber auch eine Schutzschicht mit aufgerollt werden.

Bei Schnellverbänden erfolgt die Herstellung indem aus dem beschichteten Träger die gewünschte Breite zugeschnitten und mit einem Wundkissen sowie einer Abdeckfolie versehen wird. Für Schnellverbände "am Meter", welche vom Anwender selbst zugeschnitten werden, wird dazu ein Vlies oder ein anderes als Wundkissen geeignetes Material durchgehend klebstoffseitig auf den Träger gelegt und darauf eine vorzugsweise zweiteilige Schutzfolie aufgebracht. Dann wird dieses Band auf die gewünschte Länge zugeschnitten. Für Schnellverbände in einer speziellen Form (z.B. Strips) werden einzelne Wundkissen in geeignetem Abstand zu einander klebstoffseitig auf den Träger und darauf wiederum eine vorzugsweise zweiteilige Schutzfolie gelegt. Daraus werden dann die gewünschten Formen ausgestanzt. Auch bei der Anwendung eines solchen Systems wird zuerst die Schutzfolie entfernt und dann der mit der Klebstoffschicht und dem Verbandstoff (Vlies oder Gaze) versehene Träger klebstoffseitig auf die Haut aufgebracht.

Der Grad der Trocknung kann vom Verwendungszweck abhängen und entweder vollständig sein oder aber einen Restgehalt an Lösungsmittel, insbesondere Wasser, vorsehen. Ein solcher Restgehalt an Lösungsmittel kann z.B. vorteilhaft für die Wirkstoffabgabe in resp. die Wirkstoffaufnahme durch die Haut sein.

Die Poly(meth)acrylatdispersion weist üblicherweise einen Feststoffanteil von 40 bis 70 Gew.-% auf. Geeignete Lösungsmittel sind neben Wasser insbesondere Alkohole, wobei Wasser aus Umweltschutzgründen bevorzugt ist.

Die Erfindung wird nun anhand von Beispielen, welche die Erfindung in keiner Weise einschränken sollen, näher erläutert.

### Beispiele

### Verwendete Stoffe

Acronal und Collacral sind Marken der BASF und die entsprechenden Produkte von dieser erhältlich.

### Beispiel 1: Hautpflegendes Vliespflaster

| | | |
|---|---|---|
| 2000g | Klebmasse "D-205" | 1910g Acronal V 205 D (69%) ca. 68g NaOH 15%, bis pH der Klebmasse 5.9 - 6.1 22g Collacral VAL (30%) |
| 60g | Mandelöl | |

Das hautpflegende Öl wird mit einem Dispergiergerät unter die Klebmasse gemischt, bis eine homogene Dispersion entsteht. Die Masse wird so auf ein Vlies beschichtet, dass das Trockengewicht der Klebmasse zwischen 100 und 140g/m² liegt. Die Klebmasse wird bei ansteigenden Temperaturen von 70-155°C getrocknet. Das Vlies wird auf die gewünschte Breite zugeschnitten und aufgerollt. Bei Bedarf kann eine Schutzfolie mit aufgerollt werden.

### Beispiel 2: Hautpflegender Schnellverband

Ein wie in Beispiel 1 beschrieben beschichtetes Vlies wird auf die gewünschte Breite zugeschnitten, ein Wundkissen wird aufgebracht und mit einer Schutzfolie zugedeckt. Dieses Band wird danach auf die gewünschte Länge zugeschnitten.

### Beispiel 3: Wirkstoffhaltiges Pflaster

| | | |
|---|---|---|
| 2000g | Klebemasse "D-85" | 1892g Acronal 85 D (55%) ca. 58g NaOH 15%, bis pH der Klebmasse 5.9 - 6.1 50g Collacral VAL (30%) |
| 52,8g | Mandelöl | |
| 21,2g | Rosmarinöl | |
| 21,2g | Lavendelöl | |
| 15,6g | Cayennepfefferextrakt | |

Die Öle werden mit einem Dispergiergerät unter die Klebmasse gemischt, bis eine homogene Dispersion entsteht. Die Masse wird dann in einer Schichtdicke von ca. 0,3 mm auf Silikonpapier beschichtet, mit einem Vlies abgedeckt und durch eine Walze in dieses hineingedrückt. Die Masse wird bei Temperaturen bis 85°C getrocknet, dabei verbindet sie sich mit dem Vlies.

Infolge der Anwesenheit der Capsaicinoide (Wirkstoffe aus dem Cayennepfefferextrakt) insbesondere zusammen mit dem durchblutungsfördernden Rosmarinöl, eignet sich ein solches Pflaster zur Behandlung von Schmerzen infolge Weichteilrheumatismus, degenerativer Erkrankungen oder nach traumatischen Läsionen.

### Beispiel 4: Wirkstoffhaltiges Pflaster

| | | |
|---|---|---|
| 3000g | Klebemasse "D-85" | 2838g Acronal 85 D (55%) ca. 87g NaOH 15%, bis pH der Klebmasse 5.9-6.1 75g Collacral VAL (30%) |
| 158,4g | Prolin (gesiebt) | |
| 79,2g | Mandelöl | |
| 15,8g | Ölsäure | |
| 201,2g | Pfefferminzöl | |

Alle Bestandteile werden mit einem Dispergiergerät gemischt, bis eine homogene Dispersion entsteht. Die Masse wird dann in einer Schichtdicke von ca. 0,3 mm auf Silikonpapier beschichtet, mit einem Vlies abgedeckt und bei Temperaturen bis 100°C getrocknet.

Ein solches prolinhaltiges Pflaster eignet sich zur Behandlung von Schmerzen infolge degenerativer Erkrankungen.

## Patentansprüche

1. Klebstoffzusammensetzung, **dadurch gekennzeichnet, dass** sie ein Poly(meth)acrylat und mindestens ein fettes Öl enthält oder daraus besteht.

2. Klebstoffzusammensetzung gemäss Anspruch 1, **dadurch gekennzeichnet, dass** das Öl Mandelöl oder mindestens eine seiner Komponenten ist oder enthält.

3. Klebstoffzusammensetzung gemäss Anspruch 2, **dadurch gekennzeichnet, dass** das Öl Mandelöl ist.

4. Klebstoffzusammensetzung gemäss einem der vorangehenden Ansprüche, **dadurch gekennzeichnet, dass** das Öl in einer Menge von max. 10 Gew.-% bezogen auf das Poly(meth)acrylat vorhanden ist.

5. Klebstoffzusammensetzung gemäss einem der vorangehenden Ansprüche, **dadurch gekennzeichnet, dass** das Öl in einer Menge von 3 bis 6 Gew.-% bezogen auf das Poly(meth)acrylat vorhanden ist.

6. Klebstoffzusammensetzung gemäss einem der vorangehenden Ansprüche, **dadurch gekennzeichnet, dass** das Poly(meth)acrylat ausgewählt ist aus carboxylgruppenhaltigen Acrylsäureestercopolymeren oder Polyacrylsäurealkylestern, insbesondere Polyacrylsäurebutylestern.

7. Klebstoffzusammensetzung gemäss einem der Ansprüche 1 bis 6, **dadurch gekennzeichnet, dass** sie zusätzlich mindestes einen Wirkstoff und gegebenenfalls mindestens einen die Wirkstoffabgabe und/oder die Wirkstoffpenetration fördernden Stoff enthält.

8. Klebstoffzusammensetzung gemäss einem der vorangehenden Ansprüche, **dadurch gekennzeichnet, dass** die Klebstoffzusammensetzung zusätzlich ein Lösungsmittel enthält und in Form einer Dispersion vorliegt.

9. Klebstoffzusammensetzung gemäss Anspruch 8, **dadurch gekennzeichnet, dass** das Lösungsmittel ausgewählt ist aus der Gruppe umfassend Ethanol, Wasser und Mischungen derselben.

10. Klebstoffzusammensetzung gemäss Anspruch 9, **dadurch gekennzeichnet, dass** das Lösungsmittel Wasser ist.

11. Klebstoffzusammensetzung gemäss einem der Ansprüche 1 bis 7, **dadurch gekennzeichnet, dass** sie max. ca. 5 Gew.-% Lösungsmittel enhält oder lösungsmittelfrei ist.

12. Verfahren zur Herstellung einer Klebstoffzusammensetzung gemäss einem der Ansprüche 1 bis 10, **dadurch gekennzeichnet, dass** einer Poly(meth)acrylatdispersion mindestens ein fettes Öl zugemischt wird.

13. Verfahren zur Herstellung einer Klebstoffzusammensetzung gemäss Anspruch 11, **dadurch gekennzeichnet, dass** einer Poly(meth)acrylatdispersion mindestens ein fettes Öl zugemischt wird, und dass die resultierende Mischung anschliessend bis zum gewünschten Restlösungsmittelgehalt von max. ca. 5 Gew.-% getrocknet wird.

14. Verbandmaterial oder transdermales therapeutisches System, **dadurch gekennzeichnet, dass** es ein Trägermaterial und eine Klebstoffzusammensetzung gemäss Anspruch 11 aufweist.

15. Verbandmaterial oder transdermales therapeutisches System gemäss Anspruch 14, **dadurch gekennzeichnet, dass** das Trägermaterial ausgewählt ist aus der Gruppe bestehend aus Gewebe, Gewirke, wasserabstossender Folie und insbesondere Vlies.

16. Verbandmaterial oder transdermales therapeutisches System gemäss Anspruch 14 oder 15, **dadurch gekennzeichnet, dass** das Trägermaterial ein mit elastischen Fäden durchwirktes Material ist.

17. Verfahren zur Herstellung eines Verbandmaterials oder eines transdermalen therapeutischen Systems gemäss einem der Ansprüche 14 bis 16, **dadurch gekennzeichnet, dass** auf einen Träger resp. ein Trägermaterial direkt eine Klebstoffzusammensetzung nach einem der Ansprüche 8 bis 10 aufgetragen und anschliessend bis zu einem Lösungsmittelgehalt, insbesondere einem Wassergehalt von max. ca. 5 Gew.-% oder vollständig getrocknet wird.

18. Verfahren zur Herstellung eines Verbandmaterials oder eines transdermalen therapeutischen Systems gemäss einem der Ansprüche 14 bis 16 im Transferverfahren, **dadurch gekennzeichnet, dass** auf eine Folie oder ein silikonisiertes Papier eine Klebstoffzusammensetzung nach einem der Ansprüche 8 bis 10 aufgetragen wird, dass vor der Trocknung ein Trägermaterial auf die Klebstoffschicht aufgebracht wird und dass anschliessend bis zu einem Lösungsmittelgehalt, insbesondere einem Wassergehalt von max. ca. 5 Gew.-% oder vollständig getrocknet wird.

19. Verfahren gemäss Anspruch 17 oder 18, **dadurch gekennzeichnet, dass** die Klebstoffzusammensetzung in einer Menge von oder entsprechend ca. 500 bis 600 g/m² bei einem Feststoffgehalt von 50 bis 55 Gew.-% aufgebracht wird.

20. Verfahren gemäss Anspruch 17 oder 18, **dadurch gekennzeichnet, dass** die Klebstoffzusammensetzung in einer Menge von oder entsprechend ca. 100 bis 250 g/m² bei einem Feststoffgehalt von 50 bis 55 Gew.-% aufgebracht wird.

21. Verfahren gemäss einem der Ansprüche 17 bis 20, **dadurch gekennzeichnet, dass** die Trocknung bei 85 bis 150°C, insbesondere mittels Heissluft, erfolgt.

22. Schnellverband, **dadurch gekennzeichnet, dass** ein Verbandmaterial nach einem der Ansprüche 14 bis 16 klebstoffseitig teilweise mit einem auf dem Klebstoff haftenden Verbandstoff, insbesondere einem Vlies, versehen ist.
